Europäisches Patentamt

European Patent Office

Office européen des brevets

(19)

(11) Veröffentlichungsnummer: **0 306 024**
**A2**

(12) **EUROPÄISCHE PATENTANMELDUNG**

(21) Anmeldenummer: **88114284.8**

(22) Anmeldetag: **01.09.88**

(51) Int. Cl.⁴: **A61F 2/38**

(30) Priorität: **04.09.87 CS 6431/87**

(43) Veröffentlichungstag der Anmeldung:
**08.03.89 Patentblatt 89/10**

(84) Benannte Vertragsstaaten:
**AT CH DE FR GB IT LI SE**

(71) Anmelder: **SPOFA Spojené Podniky Pro Zdravotnickou Vyrobu**
**Husinecká 11 a**
**Prag 3(CS)**

(72) Erfinder: **Cerny, Pavel, Dipl.-Ing.**
**Klimentská 36**
**Praha 1(CS)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

(54) **Künstliches Kniegelenk.**

(57) Gegenstand der Erfindung ist ein künstliches zweiachsiges Kniegelenk mit einem nicht linearen Übersetzungsverhältnis, das konstruktiv einfach aufgebaut ist und kostengünstig hergestellt werden kann. Je ein stabiler Schienenarm 5, 6 ist mit dem Oberschenkel bzw. mit dem Unterschenkel verbunden und gelenkig zwischen Seitenwänden 4 gelagert. Die Übersetzung zwischen den Schienenarmen 5, 6 wird durch einen Bolzen 8 und eine Nut 10 gebildet.

FIG. 2

EP 0 306 024 A2

## Künstliches Kniegelenk

Die Erfindung betrifft ein zweiachsiges Kniegelenk der im Oberbegriff des Patentanspruchs 1 angegebenen Gattung.

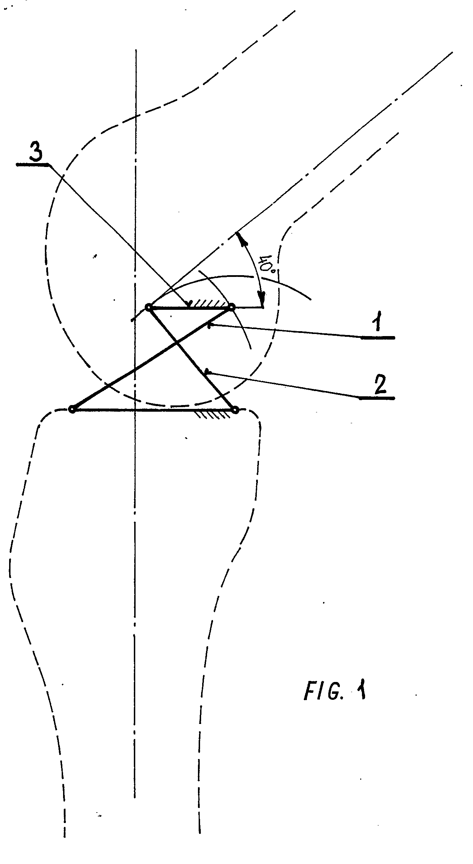

Bekannte orthopädische Kniegelenke enthalten meist ein Zahnradgetriebe mit einem linearen Übersetzungsverhältnis. Ihre Einzelteile bestehen aus Edelstahl bzw. Titan und aus Kunststoffen. Das Zahnradgetriebe ermöglicht physiologische Bewegungen in einem Schwenkbereich von etwa 0 bis 90° mit minimalen Abweichungen, wie sie bei dem Gelenkschema in Fig. 1 angedeutet sind. Bei Beugungswinkeln von 90° bis 140° vergrößern sich allmählich die Abweichungen bzw. Differenzen. Ein Nachteil derartiger Kniegelenke mit Zahnradgetriebe liegt in der relativ komplizierten Herstellung. Die aus Kunststoff gespritzten Gelenkteile erfordern komplizierte Spritzformen und die Metallteile mit der Verzahnung müssen in vielen gesonderten Arbeitsgängen mit höchster Präzision hergestellt werden. Ferner unterliegen die Gelenkteile einem gewissen Verschleiß, so daß ihre Lebensdauer begrenzt ist.

Aufgabe der Erfindung ist es, ein künstliches Kniegelenk zu schaffen, das im erweiterten Beugebereich einen dem natürlichen Gelenk entsprechenden Bewegungsablauf ermöglicht, das konstruktiv einfach ohne Verzahnung aufgebaut ist und eine lange Lebensdauer hat.

Diese Aufgabe wird erfindungsgemäß durch die im Patentanspruch 1 angegebenen Merkmale gelöst.

Das erfindungsgemäße zweiachsige Kniegelenk mit einem nicht linearen Übersetzungsverhältnis besitzt einen in einem oberen Schienenarm befestigten Zapfen, der in einer im unteren Schienenarm eingearbeitete schräge Nut geführt ist. Die beiden Schienenarme mit Distanzunterlagen sind auf Bolzen drehbar gelagert, welche in zwei Seitenwänden fest verankert sind. Das Übersetzungsverhältnis bei der Beugung der beiden Schienenarme ändert sich kontinuierlich. Alle Bauteile des erfindungsgemäßen Gelenkes haben einfache Formen und können daher kostengünstig aus Metall oder auch aus Kunststoff hergestellt werden.

Ein weiterer Vorteil liegt in der Möglichkeit einer gezielten Änderung des Bewegungsverlaufes durch eine spezifische Anordnung des Bolzens und der Nut, der Nutneigung und auch der Nutform. Drehende Bauteile mit Verzahnungen werden beim erfindungsgemäßen Gelenk nicht benötigt.

Weitere Merkmale und Vorteile der Erfindung ergeben sich aus der folgenden Beschreibung und aus der beiliegenden Darstellung von Ausführungsbeispielen.

In der Zeichnung zeigen:

Fig. 1 ein physiologisches Kniegelenkschema,

Fig. 2 schematisch eine Ausführung des erfindungsgemäßen zweiachsigen Kniegelenkes mit dem Oberschenkel-Knochen und dem Schienbein, das physiologische Bewegungen nur mit geringfügigen Abweichungen ausführt,

Fig. 3 das kinematische Schema des erfindungsgemäßen zweiachsigen Kniegelenkes,

Fig. 4 eine Seitenansicht einer konkreten Kniegelenkausführung,

Fig. 5 einen zentralen Vertikalschnitt der Ausführung nach Fig. 4.

Fig. 1 zeigt einen Viergelenkmechanismus mit Armen 1, 2, 3 in Analogie zu einem anatomisch aufgebautem natürlichen Kniegelenk. Der Arm 3 ist mit dem Oberschenkel-Knochen fest verbunden und schließt mit dessen gestrichelter Längsachse einen Winkel von 40° ein. Die Arme 1 und 2 sind auf dem Schienbein drehbar gelagert.

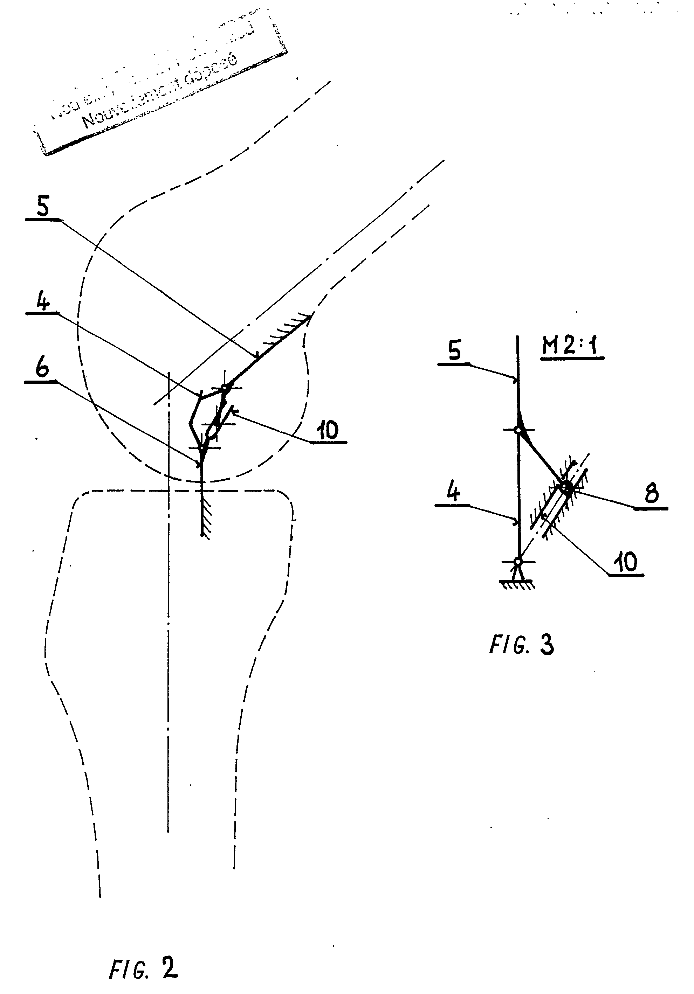

Die Anordnung und kinematische Verknüpfung der Einzelteile des erfindungsgemäßen zweiachsigen Kniegelenkes ist den Fig. 2 und 3 entnehmbar. Ein in der dargestellten Weise geformter unterer Schienenarm 6 ist mit dem gestrichelt angedeuteten Schienbein fest verbunden und weist in seinem oberen Endteil eine schräge Nut auf. Ein oberer Schienenarm 5 ist mit dem Oberschenkel-Knochen fest verbunden und trägt einen Zapfen 8, der in der Nut 10 verschiebbar geführt ist. Eine Seitenwand 4 ist am Oberschenkel-Knochen und am Schienbein gelagert.

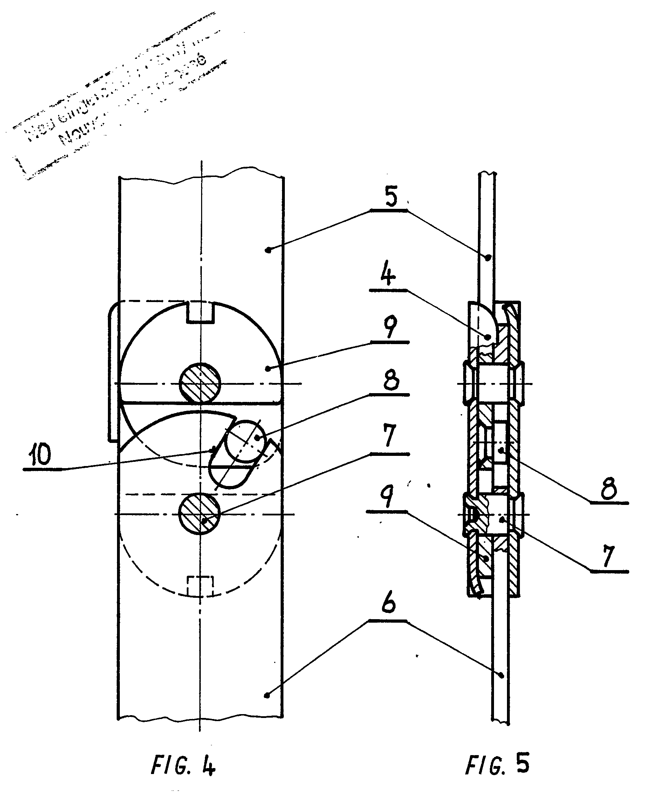

Die in Fig. 4 und 5 dargestellte konkrete Ausführung des erfindungsgemäßen Kniegelenkes mit nicht linearem Übersetzungsverhältnis entspricht in etwa dem Modell nach Fig. 2. In zwei Seitenwänden 4 sind durch Vernietung zwei Gelenkbolzen 7 befestigt, an denen die Schienenarme 5 und 6 drehbar gelagert sind. In dem oberen Schienenarm 5 ist der Zapfen 8 außermittig zur Längsachse durch Vernietung befestigt, der in die in dem unteren Schienenarm 6 geradlinig ausgebildete Schrägnut 10 eingreift. Die Schienenarme 5 und 6 überlappen einander, wobei die entstehenden Zwischenräume durch Distanzunterlagen 9 ausgefüllt sind. Eine in den Seitenwänden 4 ausgebildete Durchpressung greift in Einschnitte in den Distanzunterlagen 9 ein, um ihre unerwünschte Verdrehung bei der Gelenkbewegung zu verhindern. An einem Abschnitt einer Seitenwand 4. an welchem der obere Schienenarm 5 anliegt, ist ein Begrenzungsanschlag durch Umbiegen ihres Seitenrands ausgebildet. Die Seitenwände 4 und die Schienenarme 5 und 6 bestehen aus Edelstahl, die Gelenkbolzen 7 und 8 aus rostfreiem Stabstahl und die Distanzunterlagen 9 aus Kunststoff, wie z. B. Polyvinylchlorid, Polyamid od. dgl. Bei dem dargestellten Beispiel ist die Nut 10 unter 45° radial zum Gelenkbolzen 7 des unteren Schienenarms 6 ausgerichtet und der Führungszapfen ist am unteren Rand des oberen Schienenarms 5 seitlich neben der strichpunktierten Längsachse angeordnet. Die schwenkbare Lagerung der beiden mehrfach abgewinkelten aus ggf. Flachstahl bestehenden Schienenarme 5, 6 zwischen den beiden Seitenwänden 4 in Verbindung mit der gemeinsamen Zwangsführung ihrer sich überlappenden Endteile ergibt das gewünschte, nicht lineare bzw. veränderliche Über-setzungsverhältnis während des vollen Bewe-gungsablaufs.

6) aus ggf. geformtem rostfreiem Flachstahl bestehen, wobei in den freien Zwischenräumen der Gelenkverbindung Distanzunterlagen (9) aus einem geeigneten Kunststoff angeordnet sind.

## Ansprüche

1. Künstliches Kniegelenk, bestehend aus mindestens einem am Oberschenkel verankerten hochfesten Bauteil, mindestens einem am Unterschenkel verankerten hochfesten Bauteil und aus Gelenkverbindungen beider Bauteile mit nicht linearer Übersetzung zumindest innerhalb bestimmter Beugewinkelbereiche,
**dadurch gekennzeichnet,**
daß die beiden Bauteile als Schienenarme (5, 6) ausgeführt sind, deren sich überlappende Enden zwischen Seitenwänden (4) um je einen Zapfen (7) schwenkbar gelagert sind, und
daß die sich überlappenden Enden der beiden Schienenarme (5, 6) aneinander zwangsgeführt sind.

2. Kniegelenk nach Anspruch 1,
**dadurch gekennzeichnet,**
daß im Endteil des einen Schienenarmes (6) eine Nut (10) und am Endteil des anderen Schienenteils (5) ein in der Nut (10) geführter Zapfen (8) vorgesehen sind, die zusammen die Zwangsführung bilden.

3. Kniegelenk nach Anspruch 2,
**dadurch gekennzeichnet,**
daß die im unteren Schienenarm (6) ausgebildete Nut (10) geradlinig unter einem spitzen Winkel zur Längsachse des Schienenarms (5) verläuft und daß der Zapfen (8) seitlich neben der Längsachse am unteren Rand des oberen Schienenarms (5) angeordnet ist.

4. Kniegelenk nach einem der Ansprüche 1 - 3,
**dadurch gekennzeichnet,**
daß an zumindest einer der Seitenwände (4) ein Begrenzungsanschlag für die Auslenkbewegung zumindest eines der Schienenarme (5, 6) vorgesehen ist.

5. kniegelenk nach einem der Ansprüche 1 - 4,
**dadurch gekennzeichnet,**
daß die Seitenwände (4) und die Schienenarme (5,

FIG. 1

5

4

6

10

**FIG. 2**

5

**M 2:1**

4

8

10

**FIG. 3**

5

4

9

8

10

7

9

6

FIG. 4

8

7

FIG. 5